Europäisches Patentamt

European Patent Office    (11) Publication number: **0 035 047**

Office européen des brevets                              **B1**

(12)            **EUROPEAN PATENT SPECIFICATION**

(45) Date of publication of patent specification: **25.01.84**    (51) Int. Cl.³: **C 07 C 103/52** //A23L1/236

(21) Application number: **80101064.6**

(22) Date of filing: **03.03.80**

(54) Process for the production of an alpha-L-aspartyl-L-phenylalanine lower alkyl ester and an intermediate therefor.

(43) Date of publication of application:
**09.09.81 Bulletin 81/36**

(45) Publication of the grant of the patent:
**25.01.84 Bulletin 84/4**

(84) Designated Contracting States:
**BE CH DE FR GB IT NL**

(56) References cited:
**AU - B - 457 103**
**GB - A - 1 243 169**
**US - A - 3 808 190**
**US - A - 3 879 372**

(73) Proprietor: **AJINOMOTO CO., INC.**
**5-8, Kyobashi 1-chome, Chuo-ku**
**Tokyo 104 (JP)**

(72) Inventor: **Satoji, Takahashi**
**958, Kashimada Saiwai-ku**
**Kawasaki-shi Kanagawa-Ken (JP)**
Inventor: **Toi, Koji**
**1642-100, Nagae, Hayamamachi**
**Miuragun Kanagawa-Ken (JP)**

(74) Representative: **Schübel-Hopf, Ursula et al,**
**Strehl, Schübel-Hopf, Schulz Patentanwälte**
**Widenmayerstrasse 17**
**D-8000 München 22 (DE)**

Courier Press, Leamington Spa, England.

Process for the production of an $\alpha$-L-aspartyl-L-phenylalanine lower alkyl ester
and an intermediate therefor.

This invention relates to a process for producing an $\alpha$-L-aspartyl-L-phenylalanine lower alkyl ester.

An $\alpha$-L-aspartyl-L-phenylalanine lower alkyl ester is a new excellent sweetener possessing a strongly sweet sugar-like taste.

Heretofor, there have been proposed several methods for synthesis of the compound. According to one method, the $\alpha$-carboxyl group of L-aspartic acid whose amino and $\beta$-carboxyl groups are both protected is first converted to a very reactive ester, the ester is then condensed with L-phenylalanine methyl ester, and the protecting groups are finally removed. See Dutch published unexamined patent application No. 6800870. This method is, however, industrially disadvantageous, because it involves many reaction steps and needs many kinds of auxiliary raw materials. There is another method in which L-aspartic acid, whose amino group is protected by benzyloxycarbonyl group, is first dehydrated to L-N-benzyloxycarbonyl-aspartic acid anhydride, the anhydride is then condensed with L-phenylalanine methyl ester, and the protecting group is finally removed. This second method also has some defects from the industrial point of view. For example, the protecting agent is expensive and a dangerous reductive reaction is applied to remove the protecting group. There is a third method in which L-aspartic acid is first converted to its anhydride hydrochloride, and the hydrochloride is then condensed with L-phenylalanine methyl ester. This third method is, however, industrially defective in that the removal of the by-products from the condensation reaction is very troublesome.

One of the above-discussed methods is described in British patent 1 243 169. In this patent there is disclosed a process for preparing $\alpha$-L-aspartyl-L-phenylalanine lower alkyl esters, which comprises reacting an N-protected L-aspartic anhydride, having its N-atom protected by a formyl, carbobenzoxy or p-methoxy carbobenzoxy group with an L-phenylalanine lower alkyl ester in an organic solvent and after isolation of the reaction product eliminating the protecting group. In order to produce N-carbobenzoxy-L-aspartic acid anhydride, one of the starting materials of the known process, L-aspartic acid is reacted with benzyloxycarbonyl chloride, which in turn is produced by reacting benzyl alcohol, an expensive reagent, with phosgene, which also is expensive and notoriously dangerous. The N-protected free acid then is converted into the anhydride. As already mentioned, after the isolation of the reaction product elimination of the carbobenzoxy group from the obtained N-carbobenzoxy-L-aspartyl-L-phenyl-alanine lower alkyl ester involves catalytic hydrogenation with the use of a palladium group metal as a catalyst, a process, which is rather expensive. If a p-methoxy carbobenzoxy group is used as a protecting group, the same disadvantages will appear. In case of the use of a formyl group as a protecting group the last step of the known process involves splitting off the formyl group from the N-formyl-L-aspartyl-L-phenylalanine lower alkyl ester, which is carried out under acidic conditions. This causes not only the desired splitting, but also to a certain degree hydrolysis of the ester bond. The hydrolysis causes in turn decreased yields of the desired final products.

In contrast the present process is not only free from the use of expensive reagents and dangerous reaction conditions, but also has the advantage, that the compounds forming the protecting groups may be recycled and used repeatedly. Also the reaction of splitting off the protecting group according to the present invention is carried out under mild conditions, such as by the use of a dilute acid and therefore causes no undesired side reactions.

This invention relates to an industrially advantageous new synthetic process which obviates such above-mentioned defects involved in the heretofore known methods.

Indeed, it is generally disclosed in German patent No. 1143516 that an amino acid and acetylacetone are reacted one with another in the presence of an alkali to obtain the N-(1-methyl-2-acetylvinyl) amino acid or its alkali salt, but no specific mention is made in the patent or any other publications of the preparation of an L-N-(1-methyl-2-lower aliphatic acylvinyl or 1-methyl-2-lower alkoxycarbonylvinyl)-aspartic acid from L-aspartic acid. The present inventors have, on the basis of the disclosure, found that such N-substituted L-aspartic acids or their alkali metal salts can be obtained from L-aspartic acid. They have further found that an $\alpha$-L-aspartyl-L-phenylalanine lower alkyl ester can be easily synthesized in high yields in the following way: such an N-substituted L-aspartic acid or its alkali metal salt is treated in an organic solvent with a dehydrating agent to give the corresponding acid anhydride, the anhydride is then reacted with an L-phenylalanine lower alkyl ester to give the corresponding N-substituted dipeptide lower alkyl ester, and the last-mentioned reaction product is treated with an acid to remove the N-protecting group. The present invention has been made on the basis of these findings.

It is to be noted that, though a process of this invention involves a few reaction steps, a product obtained in a reaction step may, without being isolated from the remainder of the reaction mixture resulting from the reaction step, be subjected, if accompanied by no particular disadvantages, to the following reaction step, as will be apparent from the description made hereinafter.

Examples of L-N-(1-methyl-2-lower aliphatic acylvinyl or 1-methyl-2-lower alkoxycarbonylvinyl)-aspartic acids are L-N-(1-methyl-2-acetylvinyl or propionylvinyl)-aspartic acid, and L-N-(1-methyl-2-

methoxycarbonylvinyl, ethoxycarbonylvinyl, propoxycarbonylvinyl or butoxycarbonylvinyl)-aspartic acid. These compounds may be used as such or as their salts such as alkali metal salts.

These N-substituted (i.e., N-protected) L-aspartic acids or their salts may be synthesized in the following way: L-aspartic acid is dissolved in a lower alcohol such as methanol or ethanol, together with at least an equimolecular amount of a 1,3-diketone such as acetylacetone, or methyl or ethyl acetoacetate and an alkali such as caustic soda or potash in an amount of 0.1—5, preferably 0.5—2.5, equivalents to L-aspartic acid, the resulting solution is concentrated or added with a solubility-lowering solvent such as acetone, whereby the N-substituted L-aspartic acid is crystallized as its alkali metal salt, and the alkali metal salt crystals are finally separated.

An amine such as triethylamine or triethanolamine may be used instead of a caustic alkali.

Examples of dehydrating agents are a lower fatty acid anhydride such as acetic anhydride, a lower fatty acid halide such as acetyl chloride, a phosphorous halogenide such as phosphorous trichloride, phosphorous oxygen acid halide such as phosphorous oxychloride, a sulfur oxygen acid halide such as thionyl chloride or sulfonyl chloride, and a haloformic acid ester such as ethyl chloroformate.

A dehydrating agent suffices when used in an amount sufficient to convert L-N-(1-methyl-2-lower aliphatic acylvinyl or 1-methyl-2-lower alkoxycarbonylvinyl)-aspartic acid to the inner anhydride. For example, a lower fatty acid anhydride, a lower fatty acid halide, a sulfur oxygen acid halide, or a haloformic acid ester is used in 0.5—3.0, preferably 0.8—2.0, times an amount equimolecular with the N-substituted L-aspartic acid, and a phosphorous halogenide is used in 0.5—3.0, preferably 0.8—2.0, times an amount of one mole divided by the number of the halogen atoms contained in the phosphorous halogenide molecule per mole of the N-substituted L-aspartic acid. By use of a large excess of the dehydrating agent, the desired compound can be synthesized, but by-products are also produced in large amounts. Use of a large excess of dehydrating agents is undesirable, accordingly.

Any organic solvents inert to the dehydrating agent may be used in the dehydrating treatment. Examples of such solvents are esters such as ethyl or butyl acetate, hydrocarbons such as benzene, toluene or xylene, chlorohydrocarbons such as ethylene dichloride. Dehydrating treatment may usually be carried out at temperatures between about −30°C and about 20°C, preferably between −25°C and 5°C depending on the solvent or dehydrating agent employed and other conditions. Racemization of N-substituted aspartic acid anhydride is apt to occur above 5°C. The reaction is slower below −30°C, while side reactions are apt to occur above 20°C. Shorter times suffice at higher temperatures for dehydrating treatment, while longer times are needed at lower temperatures. 5 to 25 hours suffice when the treatment is carried out between −25°C and 5°C.

A lower carboxylic acid such as formic acid or acetic acid, or a mineral acid such as hydrogen chloride or sulfuric acid, when mixed with the organic solvent, brings about some favourable effects such as improved efficiency of the dehydrating treatment and prevention of colouration. Further, as will be described hereinafter, when a solution resulting from dehydrating treatment is first reacted with an L-phenylalanine lower alkyl ester and then contacted with an acid, the aimed-at compound, i.e. the $\alpha$-L-aspartyl-L-phenylalanine lower alkyl ester, is produced in a very high yield. A lower carboxylic acid or mineral acid may be used in an amount of less than 10 moles, preferably between 1 and 5 moles per mole of L-N-(1-methyl-2 lower aliphatic acylvinyl or 1-methyl 2-lower alkoxycarbonylvinyl)-aspartic acid. It is undesirable to conduct the dehydrating reaction outside the abovementioned broader range because of lower reaction velocity and lower reaction yield.

The acid anhydride resulting from dehydrating treatment may of course be reacted with an L-phenylalanine lower alkyl ester after separated from the dehydrating treatment mixture. The reaction is carried out in the presence of an acid, organic or inorganic, whereby an $\alpha$-L-aspartyl-L-phenylalanine lower alkyl ester is increased in yields. It may also be reacted as it is contained in the mixture. In the latter case the reaction of the solution resulting from the dehydrating treatment with an L-phenylalanine lower alkyl ester may be carried out by adding to the solution resulting from the dehydrating treatment, an L-phenylalanine lower alkyl ester dissolved in such an organic solvent employable for dehydrating treatment. The reaction temperature is below 50°C. Higher temperatures above 50°C may cause side reactions. Room temperature (about 20°C) may be appropriate. A reaction time of as short as 0.5 to 2 hours suffices, because the reaction proceeds very rapidly. The L-phenylalanine lower alkyl ester is appropriately used in an amount of 0.5 to 2 moles per mole of an N-(1-methyl-2-lower aliphatic acylvinyl or 1-methyl-2-lower alkoxycarbonylvinyl)-L-aspartic acid.

When a solution resulting from reaction with an L-phenylalanine lower alkyl ester is contacted with water or preferably with an aqueous solution containing an acid, the N-protecting group is removed from the N-substituted di-peptide lower alkyl ester contained in the former solution and the formed $\alpha$- and $\beta$-L-aspartyl-L-phenylalanine lower alkyl esters separate from the organic solvent layer and move into the aqueous layer. To prepare an acid-containing solution, a mineral acid such as hydrogen chloride or sulfuric acid, or a lower carboxylic acid such as acetic acid may be used. Among these acids, hydrogen chloride is the most convenient and the most preferred concentration range is between 0.1 to 2.0 normal. Too high a concentration of an aqueous acid solution brings about undesired crystallization and/or decomposition of the aimed-at compound.

An $\alpha$-L-aspartyl-L-phenylalanine lower alkyl ester possesses a strongly sweet taste; on the other hand, a $\beta$-L-aspartyl-L-phenylalanine lower alkyl ester possesses a weakly bitter taste. Accordingly, it is desired that the former is formed in larger amounts, while the latter in smaller amounts, and that the latter is removed from the former as completely as possible.

According to this invention, an $\alpha$-L-aspartyl-L-phenyl-alanine lower alkyl ester is produced more predominantly than the corresponding $\beta$-L-aspartyl-L-phenylalanine lower alkyl ester, and the invention is very valuable from the industrial point of view.

The separation of an $\alpha$-L-aspartyl-L-phenylalanine lower alkyl ester from the $\beta$-isomer may be carried out in accordance with a known method such as recrystallization. In case of L-aspartyl-L-phenylalanine methyl ester, the $\alpha$-isomer may be separated as its hydrochloride dihydrate crystals from the last-mentioned aqueous layer, e.g. by adding concentrated hydrochloric acid to the aqueous layer. If a mixture of the $\alpha$- and $\beta$-isomers is first separated from the aqueous layer, e.g. by concentrating, the mixture is dissolved in an appropriate concentration in hydrochloric acid, $\alpha$-L-aspartyl-L-phenylalanine methyl ester hydrochloride dihydrate crystallizes out predominantly, and the purpose is sufficiently achieved by separating the crystals. $\alpha$- and $\beta$- D-aspartyl-L-phenylalanine methyl esters which may be by-produced in small amounts are removed from the $\alpha$-isomer, together with the $\beta$-isomer by these methods. The hydrochloride dihydrate may in some cases be used as much as a sweetener.

If necessary, however, the hydrochloride dihydrate crystals are dissolved in an appropriate concentration in water, and the pH of the resulting solution is adjusted with an alkali to crystallize $\alpha$-L-aspartyl-L-phenylalanine methyl ester. Highly pure $\alpha$-L-aspartyl-L-phenylalanine methyl ester may be obtained in high yields by separating the crystals—see USP 3 798 207.

As has been explained hereinabove, the process of this invention involves fewer steps and is easy to operate. In addition to these useful characteristics, an $\alpha$-L-aspartyl-L-phenylalanine lower alkyl ester, the desired compound may be obtained in high yields from L-aspartic acid, one of the main raw materials, according to the process of this invention. The process of this invention is, accordingly, an industrially excellent new process for producing an $\alpha$-L-aspartyl-L-phenylalanine lower alkyl ester, whereby savings may be made in manufacturing equipment and manufacturing cost, as compared with known methods.

L-N-(1-methyl-2-acetylvinyl)-aspartic acid dialkali-metal salt, one of the raw materials of this invention, may be prepared, for example, as under Preparation of Raw Material 1—4 below.

Preparation of raw material 1

To 133 g L-aspartic acid were added 4.0 l methanol, 118 g potassium hydroxide and 110 g acetylacetone and the mixture was stirred overnight at room temperature to give a transparent solution. The solution, after concentrated under reduced pressure to between one half and one-third of its original volume, was gradually added with 2.0 l acetone with stirring at room temperature and was allowed to stand for 5 hours. The resulting crystals were separated by filtering and dried under reduced pressure in a desiccator to give 235 g L-N-(2-methyl-2-acetylvinyl)-aspartic acid dipotassium salt.

Elemental analysis;
Calcd. for $C_9H_{10}N_1O_5K_2$: C, 37.10; H, 3.81; N, 4.81.
Found: C, 37.01, H, 3.79; N, 4.85.

A 8.775 g portion of the crystals was dissolved in 80 ml 1N (one normal) hydrochloric acid, and the solution was concentrated under reduced pressure. The residue was dissolved in 2N hydrochloric acid to obtain a solution in a total amount of 50 ml. The optical rotation of the solution was measured and calculation of the specific optical rotation of the L-aspartic acid gave $[\alpha]_D^{20}=+26.3°$. This value agrees with that of pure L-aspartic acid.

Preparation of raw material 2

To 101 g L-aspartic acid were added 1.5 l acetylacetone and 232 ml triethylamine and the mixture was stirred overnight at room temperature to give a transparent solution.

The solution was added with a solution of 85.1 g potassium hydroxide in 300 ml methynol with stirring at room temperature. 1 hour later, the resulting crystals were separated by filtration and dried in a desiccator under reduced pressure, whereby 241 g L-N-(1-methyl-2-acetylvinyl)-aspartic acid dipotassium salt was obtained.

Preparation of raw material 3

133 g L-aspartic acid, 84 g sodium hydroxide and 150 g acetylacetone were suspended in 1.3 l methanol. The mixture was heated under reflux with stirring for 2 hours.

The reaction mixture was then concentrated to about one half of its original volume and allowed to stand overnight at room temperature. The resulting crystals were separated by filtering and dried to give 194 g L-N-(1-methyl-2-acetylvinyl)-aspartic acid disodium salt.

Preparation of raw material 4

To 133 g L-aspartic acid were added 260 g ethyl acetoacetate, 250 ml triethylamine and 1.0 l methanol and the mixture was stirred overnight at room temperature to give a transparent solution. The solution was cooled to 0°C and 84 g sodium hydroxide was dissolved in it.

4

The reaction mixture was added with 2.0 l acetone. The formed crystals were separated by filtering and dried under reduced pressure in a desiccator to give 274 g L-N-(1-methyl-2-ethoxycarbonylvinyl)-aspartic acid disodium salt.

Elemental analysis;
$$\text{Calcd. for } C_{10}H_{13}NO_6Na_2: \quad C, 41.53; H, 4.53; N, 4.84.$$
$$\text{Found:} \quad C, 41.21; H, 4.48; N, 4.90.$$

A 8.71 g portion of the crystals was dissolved in 80 ml 1N (one normal) hydrochlorid acid and the solution was concentrated under reduced pressure. The residue was dissolved in 2N hydrochlorid acid to obtain a solution in a total amount of 50 ml. The optical rotation of the solution was measured and calculation of the specific rotation of the L-aspartic acid gave $[\alpha]_D^{20}=+26.4°$. This value agrees with that of pure L-aspartic acid.

The following examples are presented to further illustrate and not limit the present invention.

Example 1

To 292 g L-N-(1-methyl-2-acetylvinyl)-aspartic acid dipotassium salt were added 2.0 l ethyl acetate and 160 ml acetic acid. The mixture was cooled to −6°C and then stirred at that temperature for 30 minutes. The cooled mixture, after added with 43.7 ml phosphorous trichloride, was stirred for 10 hours.

To the reaction mixture was added a solution of 200 g L-phenylalanine methyl ester in 4 l ethyl acetate heated to 40°C. The newly resulting mixture was stirred for 30 minutes, then, after added with 1,0 l 1N (one normal) hydrochlorid acid, stirred for additional 1 hour and was allowed to stand, whereby an aqueous layer formed. The aqueous layer was separated. A small portion of it was put aside for analytical use. The remaining aqueous layer was washed by shaking with about 500 ml toluene.

The washed layer was, after cooled to 5°C, added with 150 ml concentrated hydrochlorid acid and allowed to stand overnight. The formed needle-like crystals ($\alpha$-L-aspartyl-L-phenylalanine methyl ester hydrochloride dihydrate) were separated by filtration.

The crystals were added with about 1 l water. The solution was gradually added with aqueous 10% sodium carbonate solution to adjust the pH to 3.0 and heated to 60°C. The heated solution was adjusted in pH to 4.6 with aqueous 10% sodium carbonate solution, cooled to 5°C, and allowed to stand for 5 hours. The formed needle-like crystals ($\alpha$-L-aspartyl-L-phenylalanine methyl ester hemihydrate) were separated by filtration and dried in a desiccator under reduced pressure. The dried crystals weighed 120 g and tasted strongly sweet.

Two small portions of the crystals were subjected to analysis with an automatic amino acid analyser (Column and fillings: $9\phi \times 100$ mm and Hitachi 2611 resin (a cation exchange resin) for one portion to determine $\alpha$-L-aspartyl-L-phenylalanine methyl ester, and $9\phi \times 550$ mm and Hitachi 2613 resin (a cation exchange resin) for the other to determine other substances: Eluting agent: pH 5 citric acid buffer solution; temperature: 55°C). Only $\alpha$-L-aspartyl-L-phenylalanine methyl ester was detected, but not any other substances. Measurement of the specific optical rotation of the crystals gave $[\alpha]_D^{20}=+33.7°$ (C=1, acetic acid).

Said small portion of the aqueous layer was subjected to determination of L-aspartyl-L-phenylalanine methyl ester with the same automatic amino acid analyser to reveal the $\alpha$- and $\beta$-isomers in yields of 55% and 40%, respectively, on the basis of the starting N-(1-methyl-2-acetylvinyl)-L-aspartic acid dipotassium salt.

Example 2

To 260 g L-N-(1-methyl-2-acetylvinyl)-aspartic acid disodium salt were added 500 ml ethyl acetate and 225 ml acetic acid. The mixture was cooled to −20°C and added with 125 ml acetic acid anhydride. Reaction was carried on overnight.

The reaction mixture was further treated as in Example 1. 134 g $\alpha$-L-aspartyl-L-phenylalanine methyl ester was obtained, whose specific optical rotation $[\alpha]_D^{20}$ was +33.6° (C=1, acetic acid).

Determination of L-aspartyl-L-phenylalanine methyl ester revealed the $\alpha$ and $\beta$-isomers in yields of 61% and 37%, respectively, on the basis of the starting N-(1-methyl-2-acetylvinyl)-L-aspartic acid disodium salt.

Example 3

5.18 g L-N-(1-methyl-2-acetylvinyl)-aspartic acid disodium salt was added to 50 ml ethyl acetate and the mixture was stirred and cooled to −6°C. The cooled mixture was first added with 0.87 ml phosphorous trichloride amd then 1.5 ml formic acid slowly. The reaction was continued for 10 hours. To the resulting reaction mixture was added 80 ml ethyl acetate solution heated to 35°C and containing 4 g L-phenylalanine methyl ester. Reaction was continued for 1 hour.

The newly resulting mixture was added with 200 ml 0.5 N hydrochloric acid, stirred for additional 15 minutes and was allowed to stand, whereby an aqueous layer formed. The aqueous layer was separated. The remaining organic layer was, after added with additional 200 ml 0.5 N hydrochloric acid, stirred for 15 minutes and was allowed to stand, whereby another aqueous layer formed. The aqueous layer was separated. Both the aqueous layers were combined and subjected to analysis with the same automatic amino acid analyser as used in Example 1 to determine the L-aspartyl-L-phenylalanine methyl ester. Analysis showed the $\alpha$-isomer in a yield of 54% and the $\beta$-isomer in a yield of 40% on the basis of the starting L-N-(1-methyl-2-acetylvinyl)-aspartic acid disodium salt.

Comparative experiment:

Example 3 was repeated except that no acid was added instead of 1.5 ml formic acid. Determination of the L-aspartyl-L-phenylalanine methyl ester revealed the $\alpha$- and $\beta$-isomers in yields of 18% and 10%, respectively.

Example 4

Example 3 was repeated except that 1.83 g hydrogen chloride was absorbed instead of 1.5 ml formic acid. Determination of the L-aspartyl-L-phenylalanine methyl ester revealed the $\alpha$- and $\beta$-isomers in yields of 43% and 42%, respectively.

Example 5

Example 3 was repeated except that 1.59 ml thionyl chloride was used instead of the phosphorous trichloride and the dehydrating reaction was carried out at $-15°C$. Determination of L-aspartyl-L-phenylalanine methyl ester showed the $\alpha$-isomer in a yield of 46% and the $\beta$-isomer in a yield of 38%.

Example 6

5.83 g L-N-(1-methyl-2-acetylvinyl)-aspartic acid dipotassium salt was dissolved in 50 ml acetic acid. Hydrogen chloride was bubbled through the solution, whereby KCl was precipitated. The KCl precipitate was removed by filtration to give a solution of L-N-(1-methyl-2-acetylvinyl)-aspartic acid in acetic acid. The solution was, after concentrated under reduced pressure to between one-fifth and one-sixth of its original volume, added with 10 ml toluene, cooled to $-20°C$ and added also with 2.5 ml acetic acid anhydride. The reaction was continued for 24 hours with stirring. To the resulting reaction mixture was added 80 ml toluene solution containing 5 g/dl L-phenylalanine methyl ester at room temperature.

The newly resulting reaction mixture was subjected to the hydrochloric acid treatments as in Example 3. Determination of the L-aspartyl-L-phenylalanine methyl ester showed the $\alpha$- and $\beta$-isomers in yields of 48% and 29%, respectively.

Example 7

To 5.18 g L-N-(1-methyl-2-ethoxycarbonylvinyl)-aspartic acid disodium salt were added 50 ml ethyl acetate and 3.2 ml acetic acid. The mixture was stirred and then cooled to $-8°C$. The cooled mixture, after added with 0.87 ml phosphorous trichloride, was stirred for 10 hours.

To the resulting reaction mixture was added 80 ml ethyl acetate solution heated to $35°C$ and containing 4 g L-phenylalanine methyl ester. The reaction was continued for 1 hour.

The newly resulting reaction mixture was then subjected to the same hydrochloric acid treatment as in Example 3. The determination of the L-aspartyl-L-phenylalanine methyl ester carried out in the same way as in Example 3 showed the $\alpha$- and $\beta$-isomers in yields of 54% and 39%, respectively.

Example 8

A 51.8 g portion of L-N-(1-methyl-2-acetylvinyl)-aspartic acid disodium salt obtained under Preparation of Raw Material 3 was added to a mixture of 500 ml ethyl acetate and 22.6 ml acetic acid. The new mixture was added with 9 ml phosphorous trichloride at $-6°C$ with stirring. Reaction was continued for 10 hours at the same temperature.

An about 45 ml portion of the reaction mixture was added to 25 ml methanol cooled to $-10°C$ and stirred at $-10°C$ for 5 hours, whereby all the aspartic acid moiety was methyl-esterified. The esterification reaction mixture was evaporated to dryness under reduced pressure. The residue was added with 60 ml aqueous 2N sodium hydroxide solution and allowed to stand for 2 hours at room temperature. The mixture was then added with 60 ml 2N hydrochloric acid and evaporated to dryness. The residue was made to a total volume of 100 ml by adding 2N hydrochloric acid. The concentration of aspartic acid in the solution was found to be 1.96 g/dl, when measured with an automatic amino acid analyser. The specific optical rotation of the solution $[\alpha]_D^{20}$ was found to be $+24.8°$. This value means that all the aspartic acid was in the L-form.

The remaining reaction mixture was added with about 1 l acetone cooled to $-10°C$ and, after allowed to stand for 1 hour, subjected to filtration. The filtrate was concentrated under a reduced pressure of 10 mmHg to remove 900 ml of the solvent. The residue was added with 250 ml ethyl ether cooled to $-10°C$ and the resulting oily substance was separated by decantation. The separated oily substance was dissolved in about 300 ml acetone and cooled to $-10°C$. After the insoluble materials were removed, the solution was concentrated under a reduced pressure of 10 mm Hg to remove 250 ml of the solvent. Oily substance resulted again upon addition of 200 ml ethyl ether cooled to −10°C in limited amounts to the residue and was separated by decantation. The solvent was completely removed from the separated oily substance by distillation under reduced pressure, the temperature being kept below −5°C. 12.1 g pure oily substance was obtained.

Elemental analysis;

Calcd. for $C_9H_{11}NO_4$:  C, 54.82; H, 5.62; N, 7.10.
Found:  C, 54.31; H, 5.51; N, 7.03.

The oily substance was identified as N-(1-methyl-2-acetylvinyl)-aspartic acid anhydride also by infrared absorption spectrum, nuclear magnetic resonance spectrum and ultraviolet absorption spectrum.

Example 9

To 6.98 g L-N-(1-methyl-2-n-butoxycarbonylvinyl)-aspartic acid dipotassium salt were added 50 ml ethyl acetate and 2.6 ml acetic acid. The mixture was cooled to −6°C and then stirred at that temperature for 30 minutes. The cooled mixture, after added with 0.88 ml phosphorous trichloride, was stirred 10 hours. The reaction mixture was further treated as in Example 3. Determination of the L-aspartyl-L-phenylalanine methyl ester revealed the $\alpha$- and $\beta$-isomer in yields of 56% and 39%, respectively. In this connection, the starting material, L-N-(1-methyl-2-n-butoxycarbonylvinyl)-aspartic acid dipotassium salt was prepared as follows: to 13.3 g L-aspartic acid were added 100 ml ethanol, 30.6 ml triethylamine and 19 ml n-Butyl acetoacetate and the mixture was stirred overnight at room temperature to give a transparent solution. The solution was cooled to 0°C and 12.4 g potassium hydroxide was dissolved in it. The reaction mixture, after added with 1.5 l acetone, was stirred at 10°C for 2 hours. The formed crystals were separated by filtering and dried under reduced pressure in a desiccator to give 26.6 g L-N-(1-methyl-2-n-butoxycarbonylvinyl)-aspartic acid dipotassium salt.

## Claims

1. A process for producing an $\alpha$-L-aspartyl-L-phenylalanine lower alkyl ester or its hydrochloride which comprises (a) treating an L-N-(1-methyl-2-lower aliphatic acylvinyl or 1-methyl-2-lower alkoxycarbonylvinyl)-aspartic acid or an alkali metal salt thereof with a dehydrating agent in an organic solvent containing an acid, (b) reacting the solution resulting from such treatment with an L-phenylalanine lower alkyl ester and (c) treating the resulting reaction mixture with water or an aqueous acid solution to remove the 1-methyl-2-lower aliphatic acylvinyl or 1-methyl-2-lower alkoxycarbonylvinyl group from the L-N-(1-methyl-2-lower aliphatic acylvinyl or 1-methyl-2-lower alkoxycarbonylvinyl)-aspartyl-L-phenylalanine lower alkyl ester.

2. A process for producing an $\alpha$-L-aspartyl-L-phenylalanine lower alkyl ester or its hydrochloride which comprises (a) converting an L-N-(1-methyl-2-lower aliphatic acylvinyl or 1-methyl-2-lower alkoxycarbonylvinyl)-aspartic acid or an alkali metal salt thereof to the corresponding inner anhydride with the use of a dehydrating agent in an organic solvent containing an acid, (b) reacting the anhydride with an L-phenylalanine lower alkyl ester in the presence of an acid, and (c) removing the N-protecting group (i.e. 1-methyl-2-lower aliphatic acylvinyl or 1-methyl-2-lower alkoxycarbonylvinyl group) from the N-substituted dipeptide lower alkyl ester resulting from step (b) (i.e., L-N-(1-methyl-2-lower aliphatic acylvinyl or 1-methyl-2-lower alkoxycarbonylvinyl)-aspartyl-L-phenylalanine lower alkyl ester) with the use of an acid.

3. L-N-(1-methyl-2-acetylvinyl)-aspartic acid anhydride.

## Patentansprüche

1. Verfahren zur Herstellung eines $\alpha$-L-Aspartyl-L-phenylalanin-niederalkylesters oder dessen Hydrochlorid, welches umfaßt (a) die Behandlung einer L-N-(1-Methyl-2-niederaliphatisch-acylvinyl-oder 1-Methyl-2-nieder-alkoxycarbonylvinyl)-asparaginsäure oder eines Alkalimetallsalzes dieser Verbindung mit einem Dehydratisierungsmittel in einem organischen Lösungsmittel, das eine Säure enthält, (b) Umsetzen der durch diese Behandlung gebildeten Lösung mit einem niederen Alkylester von L-Phenylalanin und (c) Behandeln des gebildeten Reaktionsgemisches mit Wasser oder einer wässrigen Säurelösung, um die 1-Methyl-2-nieder-aliphatische Acylvinyloder 1-Methyl-2-nieder-alkoxycarbonylvinyl-Gruppe von dem L-N-(1-Methyl-2-nieder-aliphatisch-acylvinyl- oder 1-Methyl-2-nieder-alkoxycarbonylvinyl)-aspartyl-L-phenylalanin-nieder-alkylester zu entfernen.

2. Verfahren zur Herstellung eines $\alpha$-L-Aspartyl-L-phenylalanin-niederalkylesters oder dessen Hydrochlorid, welches umfaßt (a) die Umwandlung einer L-N-(1-Methyl-2-nieder-aliphatisch-acylvinyl-oder 1-Methyl-2-nieder-alkoxycarbonylvinyl)-asparaginsäure oder deren Alkalimetallsalz zu dem entsprechenden inneren Anhydrid mit Hilfe eines Dehydratisierungsmittels in einem eine Säure enthaltenden organischen Lösungsmittel, (b) Umsetzen des Anhydrids mit einem niederen Alkylester von L-Phenylalanin in Gegenwart einer Säure und (c) Entfernen der N-Schutzgruppe (d.h. der 1-Methyl-2- nieder-aliphatisch-acylvinyl- oder 1-Methyl-2-nieder-alkoxycarbonylvinyl-Gruppe) von dem in Stufe (b) erhaltenen N-substituierten Dipeptid-niederalkylester (d.h. L-N-(1-Methyl-2-nieder-aliphatisch-acylvinyl- oder 1-Methyl-2-nieder-alkoxycarbonylvinyl)-aspartyl-L-phenylalanin-niederalkylester) mit Hilfe einer Säure.

3. L-N-(1-Methyl-2-acetylvinyl)-asparaginsäure-anhydrid.

**Revendications**

1. Un procédé pour la production d'un ester alkylique inférieur d'$\alpha$-L-aspartyl-L-phénylalanine ou de son chlorhydrate qui comprend (a) le traitement d'un acide L-N-(méthyl-1 acyl aliphatique inférieur-2 vinyl ou méthyl-1 alcoxycarbonyl inférieur-2 vinyl)-aspartique ou d'un de ses sels de métaux alcalins avec un agent déshydratant dans un solvant organique contenant un acide, (b) la réaction de la solution produite par ce traitement avec un ester alkylique inférieur de la L-phénylalanine et (c) le traitement du mélange réactionnel obtenu avec de l'eau ou une solution aqueuse d'acide pour éliminer le groupe méthyl-1 acyl aliphatique inférieur-2 vinyle ou méthyl-1 alcoxycarbonyl inférieur-2 vinyle de l'ester alkylique inférieur de la L-N-(méthyl-1 acyl aliphatique inférieur-2 vinyl ou méthyl-1 alcoxycarbonyl inférieur-2 vinyl)-aspartyl-L-phénylalanine.

2. Un procédé pour la production d'un ester alkylique inférieur de l'$\alpha$-L-aspartyl-L-phénylalanine ou de son chlorhydrate qui comprend (a) la conversion d'un acide L-N-(méthyl-1 acyl aliphatique inférieur-2 vinyl ou méthyl-1 alcoxycarbonyl inférieur-2 vinyl)-aspartique ou d'un de ses sels de métal alcalin en l'anhydride interne correspondant avec emploi d'un agent déshydratant dans un solvant organique contenant un acide, (b) la réaction de l'anhydride avec un ester alkylique inférieur de la L-phénylalanine en présence d'un acide et (c) l'élimination du groupe N-protecteur (c'est-à-dire du groupe méthyl-1 acyl aliphatique inférieur-2 vinyle ou méthyl-1 alcoxycarbonyl inférieur-2 vinyle) de l'ester alkylique inférieur du dipeptide N-substitué résultant du stade (b) (c'est-à-dire l'ester alkylique inférieur de la L-N-(méthyl-1 acyl aliphatique inférieur-2 vinyl ou méthyl-1 alcoxycarbonyl inférieur-2 vinyl)-aspartyl-L-phénylalanine) avec emploi d'un acide.

3. Anhydride de l'acide L-N-(méthyl-1 acétyl-2 vinyl)-aspartique.